# EUROPÄISCHE PATENTSCHRIFT

(11) **EP 0 348 814 B1**
(45) Veröffentlichungstag und Bekanntmachung des Hinweises auf die Patenterteilung: **06.04.1994**
(21) Anmeldenummer: 89111331.8
(22) Anmeldetag: 22.06.1989
(51) Int. Cl.: C12N 15/57, C12N 9/56, C12N 1/20

(54) **Hybridplasmide zur Erzeugung von Subtilisin Carlsberg in Bacillus**
Hybrid plasmid for producing subtilisin Carlsberg in bacillus
Plasmides hybrides pour produire l'enzyme subtilisine Carlsberg dans le bacillus

(30) Priorität: 25.06.1988 DE 3821491
(43) Veröffentlichungstag der Anmeldung: 03.01.1990
(73) Patentinhaber: Henkel Kommanditgesellschaft auf Aktien, 40191 Düsseldorf (DE)
(72) Erfinder: Gamon, Konrad, Dr., D-4000 Düsseldorf (DE); Berger, Harald, Dr., D-4000 Düsseldorf (DE); Hänggi, Urs, Dr., A-4020 Linz (AT); Markgraf, Martina, D-5024 Pulheim 4 (DE); Kreft, Jürgen, Dr., D-8700 Höchberg (DE)

(56) Entgegenhaltungen:
- EP-A- 0 214 435
- NUCLEIC ACIDS RESEARCH, Band 11, Nr. 22, November 1983, Seiten 7911-7925, IRL Press Ltd, Oxford, GB; J.A. WELLS et al.: "Cloning, sequencing, and secretion of Bacillus amyloliquefaciens subtilisin in Bacillus subtilis"
- NUCLEIC ACIDS RESEARCH, Band 13, Nr. 24, Dezember 1985, Seiten 8913-8926, IRL Press Ltd, Oxford, GB; M. JACOBS et al.: "Cloning, sequencing and expression of subtilisin Carlsberg from Bacillus licheniformis"
- CHEMICAL ABSTRACTS, Band 90, 4. Juni 1979, Seite 242, Zusammenfassung Nr.182109f, Columbus, Ohio, US; A.Y. STRONGIN et al.: "Direct comparison of the subtilisin-like intracellular protease of Bacillus licheniformis with the homologous enzymes of Bacillus subtilus"
- TRENDS IN BIOTECHNOLOGICAL SCIENCES, Band 13, August 1988, Seiten 291-297, Elsevier Publications, Cambridge, GB; J.A. WELLS et al.: "Subtilisin - an enzyme designed to be engineered"

## Beschreibung

Die Erfindung betrifft neue Hybridplasmide, die es ermöglichen, die alkalische Protease Substilisin Carlsberg und damit verwandte proteolytische Enzyme in erhöhten Mengen in Bacillus zu exprimieren.

Es sind zahlreiche Proteasen bekannt, die von Mikroorganismen, insbesondere Bakterien und Pilzen gebildet werden und durch aktiven Transport aus der Zelle ausgeschleust werden, so daß sie sich im Kulturmedium sammeln. Zahlreiche Enzyme dieser Art finden bereits technische Anwendung in Verfahren, bei denen es darum geht, Proteine abzubauen. Wichtige Anwendungen sind beispielsweise Wasch- und Reinigungsmittel oder auch Tiernahrungsmittel. Die Proteasen werden gängigerweise nach dem pH-Bereich eingeteilt, in dem sie ihr Wirkungsoptimum zeigen. Man spricht daher von alkalischen, neutralen und sauren Proteasen. Die alkalischen Proteasen sind von besonderer Wichtigkeit für Wasch- und Reinigungsmittel. Dabei wird eine wichtige Gruppe unter dem Oberbegriff Subtilisin zusammengefaßt. Diese Bezeichnung leitet sich von Bacillus subtilis ab, da u.a. Stämme dieser Spezies in der Lage sind, Subtilisin zu erzeugen. In den letzten Jahren ist es gelungen, verschiedene in der Natur vorkommende Arten von Subtilisin voneinander zu trennen, und strukturell aufzuklären (E.L. Smith et al, J. Biol. Chem. (1968) 243 (9), 2184). Dabei hat sich gezeigt, daß für die Praxis insbesondere zwei Arten von Subtilisin von Bedeutung sind, nämlich Subtilisin BPN′ und Subtilisin Carlsberg. Subtilisin BPN′ wird oftmals aus Bacillus amyloliquefaciens gewonnen, wo hingegen sich Subtilisin Carlsberg beispielsweise durch Kultivierung von Stämmen der Spezies Bacillus licheniformis erhalten läßt. Viele Bacillus-Stämme scheiden alkalische Proteasen oftmals im Gemisch mit neutralen Proteasen (Metallo-Proteasen) aus.

Aufgrund der technischen Bedeutung der Enzyme, die für die Protease Subtilisin Carlsberg etwas höher anzusetzen ist als für die Protease Subtilisin BPN', hat es in der Vergangenheit bereits zahlreiche Versuche gegeben, Stämme aufzufinden, die das eine oder andere Enzym produzieren und die sich zur technischen Gewinnung der Produkte eignen. Verwiesen sei beispielsweise auf die folgenden deutschen Offenlegungs- oder Patentschriften: DE 19 40 488, DE 20 18 451, DE 20 44 161, DE 21 01 803, DE 21 21 397 und DE 29 25 427 sowie auf GB 1 263 765, US 3 623 957, US 4 264 738 und EP-A 6 638. Bei den genannten Anmeldungen bzw. Patenten handelt es sich um klassische Mutationsverfahren, bei denen durch die häufige Wiederholung von Mutations- und Selektionsschritten letztendlich Produktionsstämme gewonnen werden, die im Hinblick auf Ausbeute oder Produktqualität optimiert sind. Es ist dem Fachmann bekannt, daß ein deratiger Mutationsprozeß statistisch abläuft, so daß kaum zu erwarten ist, daß dabei maßgeschneiderte Stämme entstehen, die das objektiv höchstmögliche Leistungsniveau aufweisen. Darüberhinaus können derartige Zuchtstämme auch zu Rückmutationen neigen, d.h., sie verlieren ihre günstigen Eigenschaften, zumindest teilweise und verwildern.

In der deutschen Offenlegungsschrift DE 35 27 913 werden Hybridplasmide für Mikroorganismen der Gattung Bacillus beschrieben, die eine doppelsträngige DNA enthalten, die den folgenden Aufbau aufweist:
Promotor, der von der RNA-Polymerase von Mikroorganismen der Gattung Bacillus erkannt wird, Ribosomenbindungsstelle, Startcodon, Strukturgen, das für Subtilisin Carlsberg oder dessen Teilstücke mit proteolytischer Aktivität oder Varianten mit proteolytischer Aktivität einschließlich deren Leader-Sequenzen codiert, Stopcodon, wobei an beiden Enden Oligonukleotidketten mit bis zu 400 Basenpaaren ohne spezifische Expressionsfähigkeit vorhanden sein können.

Vor dem Hintergrund dieses Standes der Technik haben sich die Erfinder die Aufgabe gestellt, besser geeignete Hybridplasmide und neue Mikroorganismen, enthaltend solche Hybridplasmide, zu schaffen, die eine noch höhere Erzeugung von Subtilisin Carlsberg bewerkstelligen können.

Gegenstand der Erfindung sind daher Hybridplasmide für Mikroorganismen der Gattung Bacillus, enthaltend ein für Bacillus geeignetes Ausgangsplasmid mit einer oder mehreren Schnittstellen für das Restriktionsenzym Ava I und gewünschtenfalls einen Resistenzmarker sowie in das Ausgangsplasmid eingefügt eine isolierte doppelsträngige DNA enthaltend:
- einen Promoter, der von der RNA-Polymerase von Mikroorganismen der Gattung Bacillus erkannt wird,
- Ribosomenbindungsstellen
- Startcodon
- Strukturgen, das nur für Subtilisin Carlsberg oder dessen Teilstücke mit proteolytischer Aktivität oder Varianten mit proteolytischer Aktivität einschließlich deren Leader-Sequenzen codiert
- Stopcodon
wobei vom Stopcodon abwärts gerechnet Oligonukleotidketten mit bis zu 400 Basenpaaren ohne spezifische Expressionsfähigkeit vorhanden sein können, dadurch gekennzeichnet, daß die eingefügte doppelsträngige DNA Promotor und Strukturgen von Subtilisin Carlsberg aus Bacillus licheniformis enthält und mit der Position -227, gerechnet vom Startcodon des Pro-/Pre-Subtilisins und gezählt bis zum Anfang der Ava I Erkennungssequenz, beginnt.

Die erfindungsgemäßen Hybridplasmide können erzeugt werden, in dem man ein Bacillus-gängiges Ausgangsplasmid, das zumindest eine und vorzugsweise nicht mehrere Ava I Schnittstellen enthält, mit einem von Ava I verschiedenen Restriktionsenzym ringöffnet, und in das geöffnete Plasmid nach an sich bekannten Methoden eine DNA einführt, die alle zur Expression von Subtilisin Carlsberg oder dessen proteolytischen Teilstücken oder Varianten nötigen Informationen und dazu eine Ava I Schnittstelle vor der Pro-/Pre-Region enthält. Anschließend wird das Plasmid wieder zum Ring geschlossen, es wird mit Ava I zweimal geschnitten und mit entsprechenden Ligationsenzymen wieder zusammengefügt.

Ganz allgemein gesprochen kann an Stelle von Ava I auch mit anderen Restriktionsenzymen gearbeitet werden. Dabei steht es dem Fachmann frei, nach den bekannten Techniken der site-directed mutagenesis geeignete Schnittstellen außerhalb der für die Expression benötigten DNA-Sequenz zu schaffen.

Die der Erfindung zugrundeliegenden Vorgänge können wissenschaftlich noch nicht erklärt werden. Zwar ist es bekannt, daß Verkleinerungen von Plasmiden eher zu einer Steigerung der Expressionsfähigkeit führen, doch war nicht zu erwarten, daß bei Entfernung eines vergleichsweise kleinen Stückes mit dem Restriktionsenzym Ava I eine derartige Erhöhung der Effizienz eintreten kann. Es wird daher angenommen, daß die zwischen den Ava I Schnittstellen gelegene und erfindungsgemäß zu entfernende DNA ein Regulationselement enthält, das der Überproduktion von Subtilisin Carlsberg in den Gaststämmen entgegenwirken kann.

Die bevorzugt in die Ausgangsplasmide einzusetzende isolierte, doppelsträngige DNA enthält die für die Expression wichtigen einzelnen Teilsequenzen in operabler Reihenfolge, d.h., die Teilsequenzen stehen in funktioneller Beziehung zueinander.
Darunter ist zu verstehen, daß ein Promotor vorhanden ist, der die Erkennungssequenz für die RNA-Polymerase besitzt und das diesem eine Bindungsstelle für die Ribosomen folgt. Es folgt dann ein in Bacillus operables Startcodon, vorzugsweise das Starttriplet ATG. Dem Starttriplet folgt dann die eigentliche Signalsequenz für das Leaderprotein und anschließend für das maturierte Subtilisin Carlsberg oder dessen Varianten bzw. Teilstücke. Unter Leadersequenz wird hier eine Prosequenz, Presequenz oder eine Pre/-Prosequenz verstanden, die vorzugsweise für die Ausschleusung des Enzyms aus der Zelle verantwortlich ist. Die Sequenz des Strukturgens ist schließlich durch ein Stopcodon begrenzt, vorzugsweise durch ein von Bacillus erkanntes Stoptriplet, wie beispielsweise TAA. Danach kann sich eine Terminatorsequenz anschließen, wobei Sequenzen bevorzugt sind, die sich in sich selber paaren können und damit zur Schleifenbildung führen (Stem-loop). Derartige Terminatorsequenzen haben die Funktion, die Synthese der messenger-RNA für das betreffende Genprodukt zu begrenzen.

An beiden Enden der geschilderten isolierten, doppelsträngigen Desoxyribonukleinsäuren können sich weitere Sequenzen anschließen, vorzugsweise solche, denen keine spezielle Expressionswirkung zugeschrieben werden kann. Diese Sequenzen sollten keine weitere Schnittstelle für Ava I aufweisen.

Die Sequenz für das maturierte Enzym kann im Rahmen der Erfindung gewissen Variationen unterworfen sein. Nach einer ersten Ausführungsform werden Sequenzen bereitgestellt, die für Subtilisin Carlsberg oder dessen Varianten kodieren. Bevorzugt sind Sequenzen, die nur aus Codons aufgebaut sind, die in Bacillus gebräuchlich sind. Nach einer bevorzugten Ausführungsform der Erfindung werden isolierte Desoxyribonukleinsäuren beansprucht, die die folgende Sequenz für das maturierte Enzym aufweisen: 
CCTCGGGACCTCTTTCCCTGCCAGGCTGAAGCGGTCTATTCATACTTTCGAACT

GAACATTTTTCTAAAACAGTTATTAATAACCAAAAAATTTTAAATTGGTCCTC

CAAAAAAATAGGCCTACCATATAATTCATTTTTTTCTATAATAAATTAACAGAATAAT

TGGAATAGATTATATTATCCTTCTATTTAAATTATTCTGAATAAAGAGGAGGAGAGTGA

GTA ATG ATG AGG AAA AAG AGT TTT TGG CTT GGG ATG CTG ACG GCC
Met Met Arg Lys Lys Ser Phe Trp Leu Gly Met Leu Thr Ala
-100

TTC ATG CTC GTG TTC ACG ATG GCA TTC AGC GAT TCC GCT TCT GCT
Phe Met Leu Val Phe Thr Met Ala Phe Ser Asp Ser Ala Ser Ala
-90 -80

GCT CAA CCG GCG AAA AAT GTT GAA AAG GAT TAT ATT GTC GGA TTT
Ala Gln Pro Ala Lys Asn Val Glu Lys Asp Tyr Ile Val Gly Phe
-70

AAG TCA GGA GTG AAA ACC GCA TCT GTC AAA AAG GAC ATC ATC AAA
Lys Ser Gly Val Lys Thr Ala Ser Val Lys Lys Asp Ile Ile Lys
-60 -50

GAG AGC GGC GGA AAA GTG GAC AAG CAG TTT AGA ATC ATC AAC GCG
Glu Ser Gly Gly Lys Val Asp Lys Gln Phe Arg Ile Ile Asn Ala
-40

GCA AAA GCG AAG CTA GAC AAA GAA GCG CTT AAG GAA GTC AAA AAT
Ala Lys Ala Lys Leu Asp Lys Glu Ala Leu Lys Glu Val Lys Asn
-30 -20

GAT CCG GAT GTC GCT TAT GTG GAA GAG GAT CAT GTG GCC CAT GCC
Asp Pro Asp Val Ala Tyr Val Glu Glu Asp His Val Ala His Ala
-10

TTG GCG CAA ACC GTT CCT TAC GGC ATT CCT CTC ATT AAA GCG GAC
Leu Ala Gln Thr Val Pro Tyr Gly Ile Pro Leu Ile Lys Ala Asp
-1 +1 10

AAA GTG CAG GCT CAA GGC TTT AAG GGA GCG AAT GTA AAA GTA GCC
Lys Val Gln Ala Gln Gly Phe Lys Gly Ala Asn Val Lys Val Ala
20

GTC CTG GAT ACA GGA ATC CAA GCT TCT CAT CCG GAC TTG AAC GTA
Val Leu Asp Thr Gly Ile Gln Ala Ser His Pro Asp Leu Asn Val
30 40

GTC GGC GGA GCA AGC TTT GTG GCT GGC GAA GCT TAT AAC ACC GAC
Val Gly Gly Ala Ser Phe Val Ala Gly Glu Ala Tyr Asn Thr Asp
50

GGC AAC GGA CAC GGC ACA CAT GTT GCC GGT ACA GTA GCT GCG CTT
Gly Asn Gly His Gly Thr His Val Ala Gly Thr Val Ala Ala Leu
60 70

GAC AAT ACA ACG GGT GTA TTA GGC GTT GCG CCA AGC GTA TCC TTG
Asp Asn Thr Thr Gly Val Leu Gly Val Ala Pro Ser Val Ser Leu
80
 
TAC GCG GTT AAA GTA CTG AAT TCA AGC GGA AGC GGA TCA TAC AGC
Tyr Ala Val Lys Val Leu Asn Ser Ser Gly Ser Gly Ser Tyr Ser
90                                      100

GGC ATT GTA AGC GGA ATC GAG TGG GCG ACA ACA AAC GGC ATG GAT
Gly Ile Val Ser Gly Ile Glu Trp Ala Thr Thr Asn Gly Met Asp
                    110

GTT ATC AAT ATG AGC CTT GGG GGA GCA TCA GGC TCG ACA GCG ATG
Val Ile Asn Met Ser Leu Gly Gly Ala Ser Gly Ser Thr Ala Met
120                                     130

AAA CAG GCA GTC GAC AAT GCA TAT GCA AGA GGG GTT GTC GTT GTA
Lys Gln Ala Val Asp Asn Ala Tyr Ala Arg Gly Val Val Val Val
                    140

GCT GCA GCA GGG AAC AGC GGA TCT TCA GGA AAC ACG AAT ACA ATT
Ala Ala Ala Gly Asn Ser Gly Ser Ser Gly Asn Thr Asn Thr Ile
150                                     160

GGC TAT CCT GCG AAA TAC GAT TCT GTC ATC GCT GTT GGT GCG GTA
Gly Tyr Pro Ala Lys Tyr Asp Ser Val Ile Ala Val Gly Ala Val
                    170

GAC TCT AAC AGC AAC AGA GCT TCA TTT TCC AGC GTC GGA GCA GAG
Asp Ser Asn Ser Asn Arg Ala Ser Phe Ser Ser Val Gly Ala Glu
180                                     190

CTT GAA GTC ATG GCT CCT GGC GCA GGC GTA TAC AGC ACT TAC CCA
Leu Glu Val Met Ala Pro Gly Ala Gly Val Tyr Ser Thr Tyr Pro
                    200

ACG AAC ACT TAT GCA ACA TTG AAC GGA ACG TCA ATG GCT TCT CCT
Thr Asn Thr Tyr Ala Thr Leu Asn Gly Thr Ser Met Ala Ser Pro
210                                     220

CAT GTA GCG GGA GCA GCA GCT TTG ATC TTG TCA AAA CAT CCG AAC
His Val Ala Gly Ala Ala Ala Leu Ile Leu Ser Lys His Pro Asn
                    230

CTT TCA GCT TCA CAA GTC CGC AAC CGT CTC TCC AGC ACG GCG ACT
Leu Ser Ala Ser Gln Val Arg Asn Arg Leu Ser Ser Thr Ala Thr
240                                     250

TAT TTG GGA AGC TCC TTC TAC TAT GGG AAA GGT CTG ATC AAT GTC
Tyr Leu Gly Ser Ser Phe Tyr Tyr Gly Lys Gly Leu Ile Asn Val
                    260

GAA GCT GCC GCT CAA TAACATATTCTAACAAATAGCATATAGAAAAAGCTAGTG
Glu Ala Ala Ala Gln
270

TTTTTAGCACTAGCTTTTTCTTCATTCTGATGAAGGTTGTTCAATATT
Diese Sequenz kodiert für ein Subtilisin Carlsberg, das in der Aminosäuresequenz in den Positionen 157 und 160 von der publizierten Aminosäuresequenz (E.L. Smith et al., J. Biol. Chem. (1968) 243 (9), 2184) abweicht.

Nach einer weiteren Ausführungsform der Erfindung können isolierte, doppelsträngige Desoxyribonukleinsäuren eingesetzt werden, die für nicht modifiziertes Subtilisin Carlsberg codieren, d.h., die anstelle der Aminosäure Serin die Aminosäure Asparagin in Position 157 aufweisen, bzw. anstelle der Aminosäure Asparagin in Position 160 die Aminosäure Serin aufweisen. Daraus ergibt sich, daß die in Bacillus gebräuchlichen Basentripletts (genetischen Codes) für diese Aminosäuren in den entsprechenden isolierten Desoxyribonukleinsäuren vorhanden sein müssen.

Die vorstehend geschilderten Desoxyribonukleinsäuren (DNA) werden in an sich bekannte Ausgangsplasmide eingesetzt, wobei diese Ausgangsplasmide eine und vorzugsweise nicht mehrere Schnittstellen für das Restriktionsenzym Ava I aufweisen müssen. Vorzugsweise enthalten die Ausgangsplasmide darüber hinaus noch einen oder mehrere Resistenzmarker.

Die Funktion des Resistenzmarkers ist darin zu sehen, daß hierdurch transformierte Mikroorganismen, die die Hybridplasmide enthalten, über ihre Resistenz von solchen unterschieden werden können, die diese Hybridplasmide nicht enthalten. Dem auf diesem Gebiet tätigen Fachmann stehen eine Vielzahl von Ausgangsplasmiden zur Verfügung. Diese verfügen über Resistenzmarker gegen beispielsweise Tetracyclin, Kanamycin, Chloramphenicol, oder andere Antibiotika. Unter den denkbaren Ausgangsplasmiden zur Herstellung der Hybridplasmide sollen solche ausgewählt werden, die für Mikroorganismen der Gattung Bacillus geeignet sind. Die erfindungsgemäßen Hybridplasmide werden daher zweckmäßigerweise aus Ausgangsplasmiden gewonnen, die ihrerseits für Bacillus geeignet sind. In der Fachliteratur ist eine Vielzahl derartiger Ausgangsplasmide beschrieben worden. Eine große Zahl geeigneter Plasmide kann bei Hinterlegungsstellen, so beispielsweise bei dem Bacillus Genetic Stock Center angefordert werden. Geeignete Ausgangsplasmide sind beispielsweise die folgenden: pBCE16, pC194, pUB110, pE194, pSA2100, pPL608, pBD64, soweit sie tatsächlich eine geeignete Ava I Schnittstelle enthalten. Hinweise auf diese Plasmide finden sich in der Fachliteratur an den folgenden Stellen:
Bernhard K., Schrempf H., Goebel W.,
1978, J. Bacteriol. 133: 897 - 903
Gryczan, T.J., Contente, S., and Dubnau, D.
1978, J. Bacteriol. 134: 318 - 329
Williams, D.M., Duvall, E.J. and Lovett, P.S.,
1981, J. Bacteriol. 146: 1162 - 1165
Gryczan, T., Shivakumar, A.G., and Dubnau, D.,
1980, J. Bacteriol 141: 246 - 253
Ehrlich, S.D., Bursztyn-Pettegrew. H., Stroynowski, J., and Leaderberg. J., 1977 In "Recombinat Molecules: Impact on Science and Society, S. 69 -80, Raven Press, New York
Weisblum, B. Graham, M.Y., Gryczan, T., and Dubnau, D. 1979, J. Bacteriol. 137: 635 - 643
Bei der Auswahl geeigneter Ausgangsplasmide ist darauf zu achten, daß sie durch spezifische Spaltenzyme linearisierbar sind, ohne dabei ihre charakteristischen Eigenschaften zu verlieren. Unter charakteristischen Eigenschaften sind hier die Replikationsfähigkeit, die Resistenz und die Möglichkeit zur Expression in Bacillus zu verstehen. Gängige spezifische Spaltenzyme, mit denen geeignete Ausgangsplasmide linearisiert werden können, sind beispielsweise Bam HI, Eco RI, Hin dIII und Pst I.

Die erfindungsgemäßen Hybridplasmide enthalten die doppelsträngigen isolierten Desoxyribonukleinsäuren, eingeschoben in eine oder mehrere derartige Spaltstellen. So kann beispielsweise das Ausgangsplasmid pBCE16, das im Sinne der Erfindung besonders geeignet ist, mit Hilfe von Bam HI linearisiert werden und dann nach Einfügung der erfindungsgemäßen doppelsträngigen Desoxyribonukleinsäuren wieder zum Ring geschlossen werden.

Die Verkleinerung des Konstrukts aus der für Subtilisin Carlsberg codierenden DNA und dem Ausgangsplasmid erfolgt, in dem man auf die Plasmide das Restriktionsenzym Ava I unter solchen Bedingungen einwirken läßt, daß sowohl entlang der Schnittstelle im Ausgangsenzym als auch entlang der Schnittstelle vor der Pre-/Pro-Region des eingeschobenen DNA-Teils gespalten wird. Die so entstandenen Enden werden mit geeigneten Enzymen religiert, beispielsweise mit T4 DNA-Ligase, wonach man die erfindungsgemäßen Hybridplasmide enthält.

Ein weiterer Gegenstand der Erfindung sind Mikroorganismenstämme der Gattung Bacillus, die Hybridplasmide nach den Ansprüchen 1 oder 2 enthalten. Zur Erzeugung bzw. Umwandlung geeigneter Mikroorganismenstämme wird der Fachmann solche Stämme auswahlen, die Hybridplasmide aufnehmen und in denen diese Hybridplasmide hinreichende Stabilität aufweisen. Bevorzugt können Stämme der Art Bacillus subtilis, Bacillus licheniformis, Bacillus amyloliquefaciens oder auch Bacillus cereus transformiert werden. Dabei können transformierte Stämme von nichttransformierten durch Detektion der gebildeten Protease z.B. auf dem nachfolgend beschriebenen immunologischen Weg unterschieden werden.

Nach einer Ausführungsform der Erfindung werden Bacillus Stämme, beispielsweise Bacillus subtilis Stämme transformiert, die nur wenig Protease bilden. Derartige Stämme sind von Hinterlegungsstellen beziehbar. Es gelingt so, Stämme zu erhalten, die nur oder zumindest überwiegend das gewünschte Subtilisin Carlsberg oder die beschriebene Variante von Subtilisin Carlsberg herstellen können. In ähnlicher Weise können jedoch auch Stämme erzeugt werden, die zusätzlich zu anderen Proteasen oder Amylasen dann Subtilisin Carlsberg ausscheiden. Namentlich ist es möglich, auf diesem Wege durch Selektion Stämme mit besonders hoher Enzymproduktion zu gewinnen.

Zur Isolierung der in die Ausgangsplasmide einzusetzenden doppelsträngigen DNA geht man bevorzugt von proteasebildenden Stämme der Art Bacillus licheniformis aus. Geeignet sind hier beispielsweise die folgenden Stämme: Bacillus licheniformis: ATCC 10716, DSM 641 sowie die Stämme DSM 3406 und DSM 3407. Diese werden gezüchtet wie in der deutschen Patentschrift 29 25 427 beschrieben. Danach werden die Zellen geerntet. Die geernteten Zellen werden der Protoplastierung unterworfen, d.h., es wird mit geeigneten Enzymen insbesondere Lysozym, das Murein-Gerüst aufgelöst und sodann mit Detergentien wie beispielsweise Natriumdodecylsulfat, die eigentliche Zellmembran aufgelöst. Die so entstandenen Lysate können zunächst einer Behandlung mit RNA-spaltenden Enzymen (RNase) oder/und Proteinase K unterzogen werden. Danach folgt der Isolierungsschritt der DNA.

Zur Isolierung der chromosomalen DNA sind dem Fachmann eine Reihe von Methoden bekannt, die entweder einzeln oder kombiniert durchgeführt werden können. Nach einer wichtigen Methode wird das Lysat zunächst einer fraktionierten Fällung in Ethanol unterzogen, wobei hochmoleklulare DNA-Stücke abgetrennt und somit isoliert werden können. Eine weitere Methode ist die Auftrennung in einem Dichtegradienten. Dazu wird in einer Cäsiumchlorid/DNA-Lösung ein Dichtegradient erzeugt. Die Gesamtmenge wird in verschiedene Fraktionen unterteilt, so daß sich die DNA in einzelnen dieser Fraktionen ansammelt, die dann abgetrennt und weiter verarbeitet werden können. In vielen Fällen ist es zweckmäßig, die Hauptmenge an Proteinen vor oder während der DNA-Isolierung durch Extraktion mit Chloroform/Isoamylalkohol-Gemisch oder in anderer Weise zu beseitigen.

Die Abtrennung der Plasmid-DNA kann grundsätzlich in gleicher Weise geschehen (Methode nach Bernhard (2)), jedoch ist es dabei zweckmäßig im Cäsiumchlorid Dichtegradienten Ethidiumbromid (roter Farbstoff, der zur Intercalation befähigt ist) einzuführen und damit künstlich einen Dichteunterschied zwischen Plasmid und Chromosomal-DNA herbeizuführen.

Die gereinigten isolierten Desoxyribonukleinsäuren werden sodann in geeignetem Puffer (bei pH-Werten zwischen 7 und 7,5) mittels Restriktionsenzymen gespalten. Geeignete Restriktionsenzyme sind beispielsweise Bam HI, Sau 3A oder andere Enzyme, die DNA an spezifischen Stellen zu spalten vermögen. Dabei kann die chromosomale DNA mit den gleichen oder auch unterschiedlichen Enzymen wie die Plasmid DNA behandelt werden. In jedem Falle hat der Fachmann darauf zu achten, daß die Spaltung der chromosomalen DNA nur unvollständig durchgeführt wird, um so Spaltstücke ausreichender Länge zu erhalten, die mit hinreichender Wahrscheinlichkeit das Strukturgen in nichtzerstörter Weise enthalten. Ferner hat erdarauf zu achten, daß die Enden der gespaltenen chromosomalen und Plasmid DNA komplementär zueinander sind, um die nachfolgende Verknüpfung mittels DNA-Ligase zu gewährleisten. Die isolierten doppelsträngigen Desoxyribonukleinsäuren werden mit den linearen Plasmiden vermischt. Dabei werden vorzugsweise die chromosomalen Desoxyribonukleinsäuren in einem hohen Gewichtsüberschuß gegenüber den linearisierten Plasmiden eingesetzt. Vorzugsweise wird ein Verhältnis von 1 : 2 bis 1 : 20, insbesondere von 1 : 5 bis 1 : 10 eingesetzt. Die gemischten Desoxyribonukleinsäuren, die dann bei einer Konzentration von etwa 0,2 mg pro ml vorliegen sollen, werden in einem darauffolgenden Schritt mittels DNA-Ligase verknüpft.

Die in die Ausgangsplasmide und insbesondere in das bevorzugte Ausgangsplasmid pBCE16 eingefügte doppelsträngige DNA enthält die Schnittstelle für das Enzym Ava I vorzugsweise vor der Pro-/Pre-Region und zwar in einer Position minus 227, gerechnet vom Startcodon des Strukturgens für die Protease Subtilisin Carlsberg einschließlich deren Leadersequenzen.

Mit dem so erhaltenen Ligationsgemisch werden kompetente Zellen von Bacillus-Arten wie Bacillus subtilis oder Bacillus licheniformis transformiert. Will man erfolgreich transformierte Stämme an ihrer Proteasebildung erkennen, so ist es bevorzugt, solche Stämme zu transformieren, die ihrerseits nicht in der Lage sind, Protease zu bilden oder wenigstens nicht in der Lage sind Subtilisin Carlsberg zu bilden. Es können jedoch auch die Ausgangsstämme transformiert werden. Um kompetente Zellen zu erhalten, wird der zur Transformation vorgesehene Stamm in einem Minimalmedium bis zur stationären Phase angezogen und danach in einem verdünnten zweiten Minimalmedium weitergezogen. Den so vorbehandelten Mikroorganismen wird meist in Pufferlösung dann das Hybridplasmid als solches angeboten. Danach kann sich eine Wachstungsphase in einem Vollmedium anschließen (Literatur Cahn, F.H. and Fox, M.S. (1968) J. Bacteriol. 95: 867 - 875). Die so erhaltenen Mikroorganismen werden sodann einem Selektionsverfahren unterworfen. Dabei wird üblicherweise zunächst in einem Medium angezüchtet, das ein Antibiotikum enthält, gegen das das Hybridplasmid Resistenz vermittelt. Auf diese Weise können in einem ersten Selektionsschritt Organismen erhalten werden, die tatsächlich das Hybridplasmid in einer oder mehreren Kopien enthalten. Diese Mikroorganismen werden dann in einem weiteren Selektionsschritt hinsichtlich ihrer Fähigkeit, Subtilisin Carlsberg zu bilden, selektioniert.

Für diesen Selektionsschritt kann vorzugsweise eine immunologische Methode eingesetzt werden. Eine geeignete immunologische Methode ist in der deutschen Patentanmeldung 35 27 913 beschrieben. Die dort geschilderte Methode basiert auf einem Verfahren von Broome und Gilbert. Hierzu werden zunächst, wie von Buckel beschrieben, Antikörper gegen Subtilisin Carlsberg durch Immunisierung von Kaninchen gewonnen. Ein Teil der Antikörper wird durch Ammoniumsulfatfällung und DEAE-Chromatographie, ein anderer Teil zusätzlich noch durch Affinitätschromatographie mit aktivierter Sepharose 4B angereichert.

Der Nachweis von Subtilisin Carlsberg kann wie folgt erbracht werden:
Eine PVC-Folie wird mit Antikörpern gegen Subtilisin Carlsberg beschichtet. Die beschichtete Folie wird auf die zu untersuchenden Bakterienkulturen gelegt. Dabei wird vorhandenes Subtilisin Carlsberg von den Antikörpern gebunden. In einem weiteren Schritt werden an den Antikörper-Subtilisin-Carlsberg-Komplex die affinitätschromatographisch gereinigten Subtilisin Carlsberg Antikörper gebunden, an die in einem vorherigen Schritt Peroxydase gekoppelt worden war.
(1) Broome, S. & Gilbert, W. (1978) Proc. Natl. Acad. Sci. USA 75, 2746 - 2749
(2) Buckel, P & Zehelein, E. (1981) Gene 16, 149 - 159
Die Detektion wird mit Tetramethylbenzidin als Peroxydase-Substrat durchgeführt. Bei Anwesenheit von Subtilisin Carlsberg positiven Klonen entwickelt sich eine blaugrüne Farbe.

Durch diese Methode ist es möglich, gezielt solche Stämme zu erkennen, die tatsächlich Subtilisin Carlsberg bilden. Dies ist besonders von Bedeutung, wenn mit Stämmen gearbeitet wird, die auch noch andere nicht auf das immunologische Verfahren ansprechende Stoffwechselprodukte aufweisen. Ein weiterer Vorteil des Detektionsverfahrens liegt darin, daß diese Arbeitsweise es ermöglicht, aus einer großen Vielzahl transformierter und nicht transformierter Stämme die Klone mit Sicherheit auswählen zu können.

Zur Konstruktion von Deletionen in der Promotorregion ist es zweckmäßig, zunächst die Vorstufe der erfindungsgemäßen Hybridplasmide in größeren Mengen herzustellen (wie vor beschrieben).

Die Vorstufen der erfindungsgemäßen Plasmide werden sodann isoliert und mit Ava I geschnitten. Anschließend kann mit T4-Ligase wieder zum Ring geschlossen werden und die DNA dann in kompetente Zellen von Bacillus-Stämmen, z.B. Bacillus subtilis oder Bacillus licheniformis transformiert werden (wie vor beschrieben).

Alternativ zur Transformation von kompetenten Zellen ist die Transformation von Protoplasten durchführbar.Dazu werden zunächst Protoplasten hergestellt, indem man die Murein-Zellwand von Mikroorganismen der Gattung Bacillus mit geeigneten Enzymen auflöst und danach die Hybridplasmide zusammen mit Polyethylenglykol auf die Protoplasten einwirken läßt. Dazu wird vorzugsweise in Pufferlösungen gearbeitet. Die Protoplasten werden dann auf einen Regenerierungsmedium weiter gezüchtet und die so erhaltenen intakten Mikroorganismen, die das erfindungsgemäße Hybridplasmid enthalten, nach den vorgenannten Selektionsverfahren von anderen Stämmen abgetrennt.

### Beispiele

### Beispiel 1

### Erzeugung der Vorprodukte der erfindungsgemäßen Hybridplasmide gemäß DE-A-35 27 913

Chromosomale DNA aus Bacillus licheniformis DSM 641 oder auch ATCC 10716 wurde nach der Methode von Marmur (1) isoliert, mit den folgenden Abwandlungen: Die Zellen wurden in 1 mg Lysozym pro ml in 10 mM Tris-hydrochlorid pH 7,5 mit 25 % Saccharose 20 Minuten in Eis inkubiert bevor EDTA und Natriumdodecylsulfat zugegeben wurde. Die Extraktion mit Chloroform-Isoamylalkohol wurde nur einmal durchgeführt.

Die DNA wurde dann 20 Stunden mit 42.000 UpM bei 20°C in einem Dichtegradienten (Dichte 1,71 g/cm³) mit Hilfe eines Vertikalrotors zentrifugiert und von anderen Zellbestandteilen abgetrennt und durch Austropfen fraktioniert.

Plasmid-DNA aus Bacillus subtilis wurde nach der Methode von Bernhard (2) isoliert, wobei die Plasmid-DNA nach der Dichtegradientenzentrifugation mittels einer Kanüle abgezogen wurde.

Als Vektorplasmid für die Klonierung in Bacillus subtilis BR 151 (3) wurde pBCE 16 (2, 4) verwendet.

Um das Gen für Subtilisin Carlsberg intakt zu erhalten, wurde eine partielle Spaltung der chromosomalen DNA von Bacillus licheniformis DSM 641 oder ATCC 10716 mit der Restriktionsendonuclease Sau 3A durchgeführt.

Da Sau 3A eine 4er Sequenz erkennt, Bam HI jedoch eine 6er-Sequenz, Sau 3A also öfter schneidet, sind die Spaltstellen für eine unvollständige Sau 3A-Spaltung gleichmäßiger verteilt als die für eine Spaltung der chromosomalen DNA mit Bam HI.

Die Spaltung wurde wie in allen folgenden Fällen entsprechend den Bedingungen des Handbuchs von Maniatis (5) durchgeführt, jedoch für die unvollständige Spaltung die Inkubationszeit, die sonst bei einer Stunde lag, entsprechend verkürzt, um Spaltprodukte zu erhalten, die schwerpunktmäßig in der Größe des Vektors pBCE 16 (ca. 3 Md) liegen. Pro 1 µg DNA wurde ca. 1 Unit Enzym eingesetzt. Der Vektor pBCE 16 wurde mit der Restriktionsendonuclease Bam HI linearisiert, das die gleichen überlappenden Enden wie Sau 3A entstehen läßt.

Nach der Inkubation wurde das Enzym zweimal mit dem halben Volumen Phenol extrahiert, dann Bam HI-gespaltenes pBCE 16 und Sau 3A-gespaltene chromosomale DNA im mengenmäßigen Verhältnis 1 : 10 gemischt, das Gemisch 5mal mit gleichem Volumen Ether extrahiert und sodann mit doppeltem Volumen Ethanol 20 Minuten bei -70°C inkubiert und die DNA durch Zentrifugation pelletiert.

Nach Trocknen wurde die DNA in folgendem Puffer gelöst: 66 mM Tris-(hydroxymethyl)-aminomethan, 5mM MgCl₂, 0,3 mM Adenosintriphosphat, 1,5 mM Dithiothreit und 0,07 mg/ml Rinderserumalbumin.

Die DNA-Konzentration wurde auf 200 ug/ml eingestellt und T4-DNA-Ligase in einer Konzentration von 1 U pro 1 µg eingesetzter DNA zugegeben. Die Ligasereaktion wurde ca. 18 Stunden bei 16°C durchgeführt. Das Ligationsgemisch, d.h. die mittels T4-DNA-Ligase verknüpften DNA-Fragmente wurden dann zur Transformation kompetenter Zellen von Bacillus subtilis BR 151 verwendet.

Kompetente Zellen von B. subtilis BR 151 wurden nach der Methode von Laird hergestellt, wie sie von Cahn und Fox (6) beschrieben wurde.

Zu 0,82 ml kompetenten Zellen wurden 0,18 ml 0,1 M MgCl₂, 0,13 ml 0,05 M CaCl₂ und 0,13 ml 100 mM EGTA (Ethylenglykol-bis-2-aminoethylether-N,N,N′,N′-tetraacetat) pH 7,3 (in dieser Reihenfolge) zugegeben und jeweils durchmischt, nach 5 Minuten leichtem Schütteln bei 30°C dann 10 µl bzw. 2 µg des Ligationsgemisches zugegeben. Nach 30 Minuten langsamen Schütteln (60 UpM) bei 30°C wurde 1 ml 2fach HGP (10 g Pepton aus Casein, 5 g Hefeextrakt, 5 g NaCl und 5 g Glucose/pro Liter) zugegeben.

Nach 90 Minuten Schütteln mit 160 UpM bei 30°C wurden die Zellen auf Calcium-Caseinat-Agar (nach Frazier und Rupp modifiziert - Merck) mit zusätzlich 0,5 % Casein und 15 µg/ml Tetracyclin zur Selektionierung der transformierten Zellen zu 100 ul pro Platte mit einem Glasspatel verteilt.

Die Platten wurden 48 Stunden bei 37°C bebrütet.

Mittels chromatographisch an Diethylaminoethylcellulose (DEAE) gereinigter Subtilisin Carlsberg-spezifischer Antikörper in Kombination mit einer von Boehringer/Mannheim lieferbaren "Test-Combination zum immunologischen Nachweis spezifischer Gen-Expression in Mikroorganismen" konnten 6 proteasepositive Klone unter insgesamt ca. 32.000 Transformanten nachgewiesen werden. Einer dieser Klone erwies sich nach mehrmaligem Überimpfen als ausreichend stabil für weitere Untersuchungen. Der Nachweis von S. Carlsberg wurde wie folgt durchgeführt:
PVC-Folien wurden zur Entfettung 2 Minuten in Isopropanol gebadet, getrocknet und 15 Folien in 40 ml 0,2 M Natriumcarbonatpuffer pH 9,2 mit 0,6 ml DEAE-gereinigte Subtilisin Carlsberg-spezifische IgG-Fraktion 10 Minuten zur Beschichtung inkubiert. Ohne Trocknung werden die Folien in ein Nachbeschichtungsbad überführt und 10 Minuten darin belassen:
40 ml PBS-Puffer⁺
+ 4,8 mg Rinder-Globulin
+ 40 mg Rinder-Serumalbumin
⁺) PBS-Puffer pH 7,5: 0,05 M KH₂PO₄; 0,1 M NaCl
Sodann wurden die Folien zwischen Papierhandtüchern getrocknet.

Die IgG beschichteten Folien wurden dann auf die bewachsenen Agarplatten aufgelegt und 2 Stunden bei Raumtemperatur inkubiert. Nach Abnehmen der Folien wurden die anhaftenden Zellen mit kaltem Leitungswasser abgespült und beidseitig mit auf 6°C temperiertem PBS-Puffer + 0,1 % Rinderserumalbumin abgespült. Ohne Trocknung wurden die Folien in 50 ml Konjugatbad (50 ml PBS-Puffer mit 0,1 % Rinderserumalbumin + 1 µl Peroxidase-konjugiertes IgG gegen Subtilisin Carlsberg) überführt und darin 4 Stunden bei Raumtemperatur belassen.

Die Folien wurden dann mit kaltem Leitungswasser abgespült und anschließend beidseitig mit 6°C temperiertem PBS-Puffer + 0,05 % Tween 20 + 0,1 % Rinderserumalbumin abgespült. Nach Trocknen zwischen Papierhandtüchern wurden die Folien auf einem Substrat (Boehringer Gen-Expressionskit^{(R)} Boehringer Mannheim Gm 611) für die gekoppelte Peroxidase inkubiert. Es handelt sich dabei um Tetramethylbenzidin, das in einer Gelatinegrundlage gelöst ist. Nach 10 bis 30 Minuten Inkubation bei Raumtemperatur entwickelt sich bei positiven Kolonien, d.h. bei Anwesenheit von Subtilisin Carlsberg, eine blaugrüne Farbe.

Die Plasmid-DNA des stabilen S. Carlsberg-positiven Klons (pC 50) wurde wie oben beschrieben isoliert.

Zur Charakterisierung der pC 50-DNA mittels verschiedener Restriktionsenzyme wurde einzeln und in Kombination mit Ava I, Bam HI, Bal I, Eco RI, Hpa II, Pst I, Sst I und Stu I gespalten.

Die Analyse der in der vertikalen Agarosegelektrophorese (7) aufgetrennten Spaltprodukte ergab folgende Plasmidkarte:
Nach Umklonierung geeigneter Fragmente des Protease-Gens aus pC50 in die Sequenzierungsvektoren pEMBL 8 bzw. pEMBL 9 (8) wurde die Sequenz nach der Kettenabbruch-Didesoxymethode (9) bestimmt.

### Gewinnung der Vorstufe der erfindungsgemäßen Hybridplasmide

Die DNA des Plasmids pC50 wurde - wie vor beschrieben - isoliert.

### Konstruktion von Deletionen in der Promotorregion und Transformierung der Konstrukte in B. subtilis

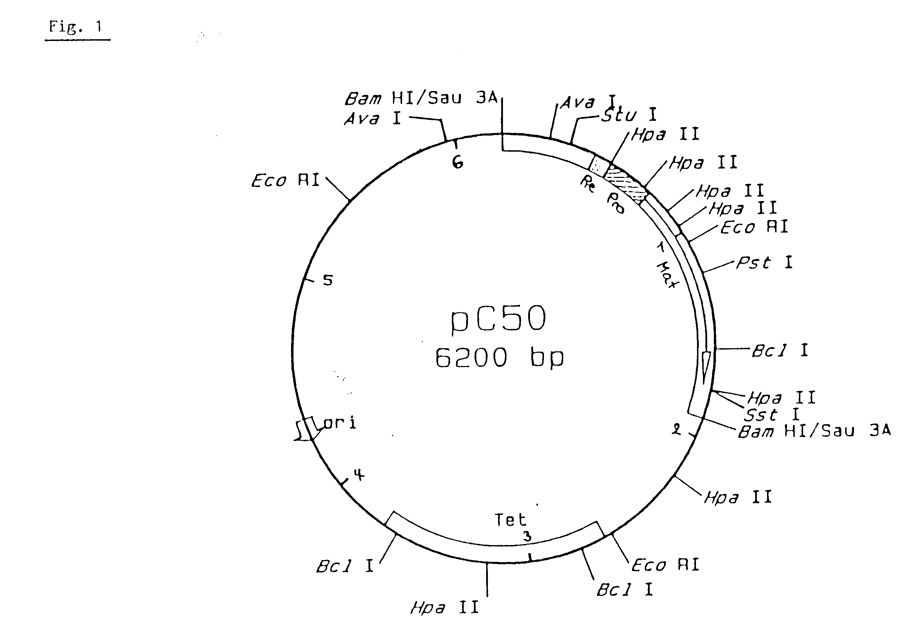

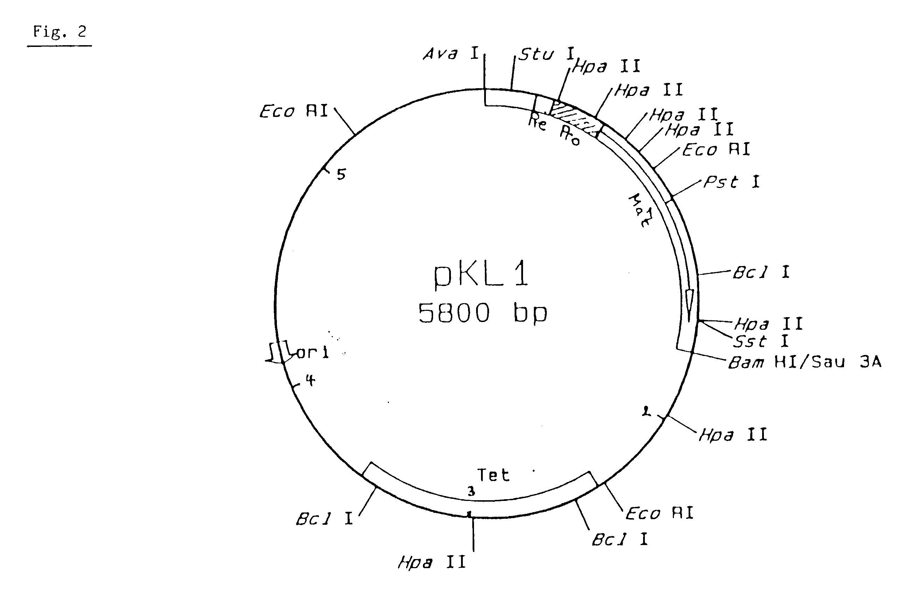

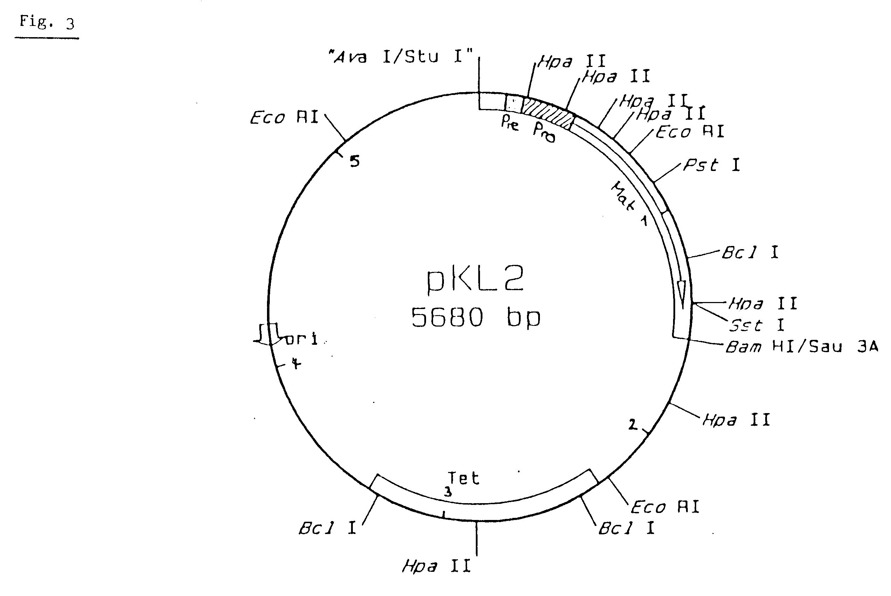

2 µg pC 50 wurden mit 6 Einheiten Ava I bzw. 6 Einheiten AVA I und 6 Einheiten Stu I geschnitten. Im Falle der Ava I/Stu I-Doppelverdauung wurden die aus der Ava I-Spaltung resultierenden überstehenden Enden durch Inkubation mit 25 mmol der vier Desoxynukleotidtriphosphate und 2 Einheiten/µg Klenow-Polymerase für 15 Minuten bei Raumtemperatur aufgefüllt. Die Polymerase wurde durch Inkubation für 10 min bei 70°C inaktiviert. Nach Religierung mit 1 Einheit/µg T4-Ligase wurde der Ligationsansatz in 200 µl kompetente Zellen von B. subtilis 202 (präpariert nach Ref. 6) transformiert. Plasmid-DNA von Tetracyclin-resistenten (7,5 µg/ml) Transformanden wurde nach der Methode von Birnboim & Doly (10) aufgearbeitet und durch Spaltung mit Eco R1 bzw. Ava I getestet. Je ein Klon wurde anhand des korrekten Spaltungsmusters ausgewählt. In pKL 1 ist das Ava I Fragment deletiert (wobei die Ava I-Schnittstelle erhalten blieb), in pKL 2 fehlt das Ava I/Stu I-Fragment (Fig. 2).

Um festzustellen, ob die Deletionskonstrukte pKL 1 und pKL2 noch für Expression des Proteasegens führen, wurden die Stämme Bacillus subtilis 202 (pC 50), 202 (pKL 1), 202 (pKL 2) und 202 (pBCE 16) als negative Kontrolle dem immunologischen Test wie vorstehend beschrieben unterzogen.

Nur im Falle von 202 (pC 50) und 202 (pKL 1) entwickelte sich die für die Anwesenheit von Subtilisin Carlsberg typische blau-grüne Farbe des Peroxidase-konjugierten Antigen-Antikörperkomplexes, während 202 (pKL 2) und 202 (pBCE 16) keine Immunreaktion zeigten.

**Tabelle 1**

| | | Reaktion mit Antikörper gegen Subtilisin Carlsberg |
|---|---|---|
| B. licheniformis DSM 641 | | + |
| B. sutilisin | 202 | - |
| " | 202 (pBCE 16) | - |
| " | 202 (pC 50) | + |
| " | 202 (pKL 1) | + |
| " | 202 (pKL 2) | - |
| Tab. 1 Qualitativer immunologischer Test | | |

Dieses Ergebnis zeigt, daß wesentliche Bereiche für die Expression von Subtilisin Carlsberg in Bacillus subtilis zwischen den Ava I- und Stu I-Schnittstellen liegen. Des erklärt auch, warum von M. Jacobs et al (11) in B. subtilis keine Expression mit einem Hybridplasmid gefunden werden konnte, das nur Protease-DNA ab der StnI-Schnittstelle in Richtung auf das Protease-Strukturgen enthielt.

Bacillus licheniformisstämme, die das Plasmid PKL 1 enthielten, zeigten gegenüber solchen, die pC 50 enthielten, im Schüttelkolben Proteasebildung in erhöhter Ausbeute.

### Literatur:

1. Marmur, J. (1961) J. Mol. Biol. 3: 208-218
2. Bernhard, K., H. Schrempf and W. Goebel (1978) J. Bacteriol. 133: 897-903
3. Bacillus Genetic Stock Center Nr. 1A40, hinterlegt in der allgemeinen Stammsammlung der Deutschen Sammlung für Mikroorganismen unter DSM 5213
4. Bacillus Genetic Stock Center Nr. 1E9
5. Maniatis, T., E.F. Fritsch, J. Sambrook (1982), Cold Spring Harbor
6. Cahn, F.H. and Fox, M.S. (1968) J. Bacteriol. 95: 867-875
7. Meyers, J., D. Sanchez, L. Elwell and S. Falkow (1975) J.Bacteriol. 127: 1529-1537
8. Dente, L., G. Casareni and R. Cortese (1983), Nucleic Acids Research 11: 1645-1655
9. F. Sanger, S. Nicklen and A.R. Coulson (1977) Proc. Nat. Acad. Sci. USA 74: 5463-5467
10. H.C. Birnboim & J. Doly (1979), Nucl. Acids Res. 7: 1513
11. M. Jacobs, M. Eliasson, M. Uhlen und J.-J. Flock (1985), Nucl. Acids Res. 13: 8913-8926.

## Patentansprüche

1. Hybridplasmide für Mikroorganismen der Gattung Bacillus, enthaltend ein für Bacillus geeignetes Ausgangsplasmid mit einer oder mehreren Schnittstellen für Restriktionsenzyme wie Ava I und gewünschtenfalls einem Resistenzmarker sowie in das Ausgangsplasmid eingefügt eine isolierte doppelsträngige DNA enthaltend:
- einen Promoter, der von der RNA-Polymerase von Mikroorganismen der Gattung Bacillus erkannt wird,
- Ribosomenbindungsstellen
- Startcodon
- Strukturgen, das nur für Substilisin Carlsberg oder dessen Teilstücke mit proteolytischer Aktivität oder Varianten mit proteolytischer Aktivität einschließlich deren Leader-Sequenzen codiert
- Stopcodon
wobei vom Stopcodon abwärts gerechnet Oligonukleotidketten mit bis zu 400 Basenpaaren ohne spezifische Expressionsfähigkeit vorhanden sein können,
dadurch gekennzeichnet, daß die eingefügte doppelsträngige DNA Promotor und Strukturgen von Subtilisin Carlsberg aus Bacillus licheniformis enthält und mit der Position -227, gerechnet vom Startcodon des Pro-/Pre-Subtilisins, beginnt.

2. Hybridplasmid nach Anspruch 1, dadurch gekennzeichnet daß die eingefügte doppelsträngige DNA Promotor und Strukturgen von Subtilisin Carlsberg aus Bacillus licheniformis, beginnend mit einer Ava I Schnittstelle vor der Pre-/Pro-Region, enthält, und daß das Hybridplasmid durch zweimaliges Schneiden mit dem Restriktionsenzym Ava I um das zwischen der Ausgangsplasmid-seitigen Ava I - Schnittstelle und einer vor der Pre-/Pro-Region der eingefügten DNA liegenden Schnittstelle liegende DNA-Stück verkleinert ist.

3. Hybridplasmide nach den Ansprüchen 1 oder 2, dadurch gekennzeichnet, daß sie sich von dem Plasmid pBCE 16 ableiten.

4. Hybridplasmid nach den Ansprüchen 1 bis 3, dadurch gekennzeichnet, daß sie zumindest einen Resistenzmarker enthalten.

5. Verfahren zur Herstellung von Hybridplasmiden nach den Ansprüchen 3 oder 4, dadurch gekennzeichnet, daß man ein Bacillus-gängiges Plasmid ringöffnet, eine isolierte DNA, enthaltend eine Ava I Schnittstelle, und die zur Erzeugung von Substilisin Carlsberg nötige Information einsetzt und sodann an beiden Ava I Schnittstellen schneidet und religiert.

6. Verfahren nach Anspruch 5, dadurch gekennzeichnet, daß man eine DNA einsetzt, die eine Ava I Schnittstelle in Position minus 227, gerechnet von Startcodon des Strukturgens einschließlich der Leadersequenzen, enthält.

7. Mikroorganismenstämme der Gattung Bacillus enthaltend Hybridplasmid nach Anspruch 1, 2, 3 und/oder 4.

8. Mikroorganismenstämme nach Anspruch 7, dadurch gekennzeichnet, daß sie zu den Arten Bacillus subtilis oder Bacillus licheniformis gehören.

9. Verfahren zur Herstellung von Subtilisin Carlsberg, dadurch gekennzeichnet, daß man Mikroorganismenstämme der Ansprüche 7 und 8 züchtet.

10. Verfahren nach Anspruch 9 dadurch gekennzeichnet, daß man ein abgewandeltes Subtilisin Carlsberg, das in der Position 157 die Aminosäure Serin anstelle der Aminosäure Asparagin aufweist und in der Position 160 Asparagin anstelle von Serin aufweist, herstellt.

## Claims

1. Hybrid plasmids for microorganisms of the genus bacillus containing a starting plasmid suitable for bacillus with one or more restriction sites for restriction enzymes, such as Ava I, and optionally a resistance marker and, inserted into the starting plasmid, an isolated double-stranded DNA containing
- a promoter which is recognized by the RNA-polymerase of microorganisms of the genus bacillus
- ribosome binding sites,
- a start codon,
- a structural gene which only codes for subtilisin Carlsberg or proteolytically active sub-units or proteolytically active variants thereof, including their leader sequences,
- a stop codon,
oligonucleotide chains containing up to 400 base pairs without any specific expression effect optionally being present from the stop codon downwards,
characterized in that the double-stranded DNA inserted contains promoter and structural gene of subtilisin Carlsberg from Bacillus licheniformis and begins at the position -227 counting from the start codon of the pro-/pre-subtilisin.

2. A hybrid plasmid as claimed in claim 1, characterized in that the double-stranded DNA inserted contains promoter and structural gene of subtilisin Carlsberg from Bacillus licheniformis beginning with an Ava I restriction site before the pre-/pro-region and in that the hybrid plasmid is reduced in size by the DNA fragment lying between the Ava I restriction site on the starting plasmid side and a restriction site preceding the pre-/pro-region of the inserted DNA by cutting twice with the restriction enzyme Ava I.

3. Hybrid plasmids as claimed in claim 1 or 2, characterized in that they are derived from the plasmid pBCE 16.

4. Hybrid plasmids as claimed in claims 1 to 3, characterized in that they contain at least one resistance marker.

5. A process for the production of the hybrid plasmids claimed in claim 3 or 4, characterized in that a plasmid suitable for bacillus is ring-opened, an isolated DNA containing an Ava I restriction site and the information required for the production of subtilisin Carlsberg is inserted and cut and religated at both Ava I restriction sites.

6. A process as claimed in claim 5, characterized in that a DNA containing an Ava I restriction site in position -227 counting from the start codon of the structural gene, including the leader sequences, is used.

7. Microorganism strains of the genus bacillus containing the hybrid plasmids claimed in claims 1, 2, 3 and/or 4.

8. Microorganism strains as claimed in claim 7, characterized in that they belong to the species Bacillus subtilis or Bacillus licheniformis.

9. A process for the production of subtilisin Carlsberg, characterized in that the microorganism strains claimed in claims 7 and 8 are cultivated.

10. A process as claimed in claim 9, characterized in that a modified subtilisin Carlsberg containing the amino acid serine instead of the amino acid asparagine in position 157 and asparagine instead of serine in position 160 is produced.

## Revendications

1. Plasmides hybrides pour des micro-organismes de l'espèce Bacillus, contenant un plasmide de base approprié pour le Bacillus avec un ou plusieurs sites de coupure pour les enzymes de restriction comme Ava I et, si souhaité, un marqueur de résistance si bien que dans le plasmide de base est inséré un ADN double brin contenant:
- un promoteur, qui est reconnu par l'ARN polymérase du micro-organisme de l'espèce Bacillus,
- des sites de liaison aux ribosomes
- un codon d'initiation
- un gène de structure, qui code pour la subtilisine Carlsberg ou une de ses sections à activité protéolytique ou une de ses variantes à activité protéolytique, y compris les séquences de tête
- un codon d'arrêt
où peuvent exister, en aval du codon d'arrêt, des chaînes d'oligonucléotides avec jusqu'à 400 paires de bases sans capacité d'expression spécifique,
caractérisés en ce que l'ADN double brin inséré contient le promoteur et le gène de structure de la subtilisine Carlsberg du Bacillus licheniformis et commence avec la position -227, calculée à partir du codon d'initiation de la pro-/présubtilisine.

2. Plasmides hybrides selon la revendication 1 caractérisés en ce que l'ADN double brin inséré contient le promoteur et le gène de structure de la subtilisine Carlsberg du Bacillus licheniformis, commençant avec un site de coupure d'Ava I de la pré-/prorégion et que le plasmide hybride en étant coupé deux fois avec l'enzyme de restriction Ava I est rapetissé d'un morceau d'ADN se trouvant entre le site de coupure d'Ava I du côté du plasmide de base et un des sites de coupure se trouvant avant la pré-/prorégion de l'ADN inséré.

3. Plasmides hybrides selon la revendication 1 ou 2, caractérisés en ce qu'ils dérivent du plasmide pCBE 16.

4. Plasmides hybrides selon les revendications 1 à 3, caractérisés en ce qu'ils contiennent au moins un marqueur de résistance.

5. Procédé de préparation des plasmides hybrides selon les revendications 3 ou 4, caractérisé en ce qu'on ouvre un anneau de plasmide habituel du Bacillus, on coupe un ADN isolé, contenant un site de coupure d'Ava I, qui détient les informations nécessaires pour la production de subtilisine Carlsberg, aux deux sites de coupure d'Ava I et ensuite on les ligature.

6. Procédé selon la revendication 5, caractérisé en ce qu'on utilise un ADN, qui contient un site de coupure d'Ava I en position moins 227, calculée à partir du codon d'initiation du gène de structure y compris la séquence de tête.

7. Souches de micro-organismes de l'espèce Bacillus contenant le plasmide hybride selon la revendication 1, 2, 3 et/ou 4.

8. Souches de micro-organismes selon la revendication 7, caractérisées en ce qu'elles concernent les souches de Bacillus subtilis ou Bacillus licheniformis.

9. Procédé de préparation de la subtilisine Carlsberg, caractérisé en ce qu'on cultive des souches de micro-organismes selon les revendications 7 et 8.

10. Procédé selon la revendication 9, caractérisé en ce qu'on prépare une subtilisine Carlsberg, qui en position 157 possède l'acide aminé sérine à la place de l'acide aminé asparagine et en position 160 l'asparagine à la place de la sérine.
